# EUROPEAN PATENT APPLICATION

(11) **EP 2 944 667 A1**
(43) Date of publication of application: **18.11.2015**
(21) Application number: 14167985.2
(22) Date of filing: 12.05.2014
(51) Int. Cl.: C08J 5/00, C01B 31/02, B82Y 30/00, B82Y 40/00, C08J 5/24

(54) **Chemically multi-functional nanomaterials for composite structures and method of production thereof**

(71) Applicant: Sabanci Üniversitesi, 34956 Istanbul (TR)
(72) Inventor: Ünal, Serkan, 34956 Istanbul (TR); Yildiz, Mehmet, 34956 Istanbul (TR)
(74) Representative: Sevinç, Erkan

(57) **Abstract**

A chemically multi-functional nanomaterial for use as binding agent in composite structures having at least two components chemically different from each other, wherein said chemically multi-functional nanomaterial is provided with a first functional group and at least a second functional group different from said first functional group, is proposed. Also two alternative methods for production of said chemically multi-functinal nanomaterial is proposed. Further, a composite structure comprising said chemically multi-functinal nanomaterial is proposed.

## Description

### Technical Field of the Invention

The present invention relates to the preparation of chemically multi-functional nanomaterials, which possess more than one type of chemically functional group wherein said functional groups are different from each other. The present invention further relates to bringing two dissimilar surfaces together by means of said chemically multi-functional nanomaterials in a composite structure.

### Background of the Invention

Hybrid composite materials composed of two or more chemically dissimilar components are designed to possess unique properties as a result of cooperative properties of all components. Due to dissimilarities between components of a composite material, special attention has to be paid to the interface.

The fiber content of fiber-reinforced polymeric composites (FRPCs) ranges between 40 to 70 wt% and premature failures in FRPCs predominantly occur at the polymer-fiber interface in FRPCs. Carbon nanomaterials such as carbon nanotubes (CNTs) are introduced to the polymer matrices for improving the performance of FRPCs, yet said high values of fiber content limits the contribution of the CNTs to the composite performance. The most common approach to incorporate CNTs into FRPCs is the pre-dispersion of CNTs in resins prior to the resin transfer molding (RTM) or the infusion process.

There are a few successful attempts found in the literature reporting the production of CNT containing FRPCs by RTM (DOI: j.carbon. 2011.06.047) or vacuum infusion processes (DOI: 10.1155/ 2010/ 453420). However, these conventional approaches fail to fully convey expected benefits of CNTs, primarily due to reasons such as increased viscosity and undesired heterogeneity of the CNT/resin dispersion, resulting in improper filling of the mold or agglomeration of CNTs on the fiber mat during the manufacturing process. These factors also significantly affect the process and limit the reproducibility of high performance composites. In fact, in this approach, mechanical performance improvement is expected solely from the mechanical reinforcement of the polymer matrix.

For the purpose of enhancing the interfacial strength between polymer matrix and the fiber surface, unique approaches such as (i) growth of CNTs on the carbon fiber by chemical vapor deposition (CVD) (DOI: 10.1063/ 1.1466880), (ii) roll-transfer (DOI: 10.1016/ j.compositesa. 2008.03.011), and (iii) treatment of carbon or glass fiber surface with CNTs by solvent-spraying (DOI: 10.1016/ j.compscitech. 2011.03.014), electrophoresis (DOI: 10.1021/ la062743p), or sizing application (DOI: 10.1016/ j.compscitech. 2010.04.010) have been reported.

Growth of CNTs on carbon fiber surface by chemical vapor deposition (CVD) process and evaluation of single fiber/epoxy composites demonstrate that nano-reinforcement has improved the interfacial load transfer, which likely is due to local stiffening of the polymer matrix as reported by Thostenson et al (DOI: 10.1063/1.1466880). Although reasonable amount of research efforts have been devoted to this approach in the literature, CVD growth of CNTs on reinforcing fibers is limited to carbon fiber surfaces, and not applicable to glass fibers. More importantly, it is industrially impractical due to the complication and cost of such process. In addition, CNTs incorporated by this method may or may not be chemically bonded only to the fiber surface, where there is certainly a lack of covalent interaction with the polymer matrix.

Alternative approaches to incorporate CNTs at the fiber-polymer matrix interface in fiber-reinforced composites were also reported as by coating glass or carbon fiber surfaces with CNTs by solvent-spraying (DOI: 10.1016/ j.compscitech. 2011.03.014) or electrophoretic deposition (DOI: 10.1021/ la062743p).

Davis et al. (DOI: 10.1016/ j.compscitech. 2011.03.014) reported a significant improvement in interfacial strength and durability of composites when amino-functionalized single-wall carbon nanotubes (SWCNTs) were deposited onto both sides of a carbon fiber fabric by a solvent spraying method, which was then fabricated by vacuum assisted RTM (VARTM). There is a lack of covalent interaction between CNTs and the fiber surface for more pronounced improvement in this study.

In another study, when a solution of acid oxidized multi-walled carbon nanotubes (MWCNTs) was sprayed onto the surface of glass fiber/epoxy prepreg, the delamination resistance of resulting composite was found to increase by at least 50% by the introduction of 1.35 wt% acid-oxidized MWCNTs, which is presumed to have only non-covalent interactions with the epoxy matrix.

Electrophoresis was also employed to deposit a uniform MWCNT film onto carbon fiber fabric, which was then fabricated into an epoxy-based composite by VARTM. The introduction of 0.27 wt% MWCNTs improved the interlaminar shear strength by 27%. The electrophoresis method reported in this study seemed to homogeneously enrich the carbon fiber surface with MWCNTs, yet an evidence for covalent attachment of MWCNTs to the fiber surface does not exist.

In none of aforementioned studies, neither a covalent bonding of CNTs to both fiber surface and polymer matrix at the interface nor an electro-spray deposition of CNTs has been considered or reported.

In addition, although there are numerous processes and studies on the chemical functionalization of carbon nanomaterials with one type of chemically functional group, there is limited number of studies in the literature on the preparation of chemically bi-functional or multi-functional carbon nanomaterials. Said studies focus on the functionalization of CNT ends asymmetrically by first embedding or aligning CNTs in a polymeric matrix or a solvent and functionalizing each end with two different chemical groups. In these studies, CNTs were reported to be asymmetrically functionalized with carboxylic acid/thiol moeities, oppositely charged ionic moeities or hydrophilic 3'-azido-3'-deoxythymidine/hydrophobic perfluorooctyl moeities. Such structures are recommended in the patent US 7 488 508 B2 for self-directed patterned or self-assembled architectures of CNTs. None of these limited studies in the literature focus on the preparation and use of bi-functional or chemically multi-functional nanomaterials in multi-component composite structures as covalently bonded nano-bridges at interfaces between each component.

### Objects of the Invention

Primary object of the present invention is to overcome the abovementioned shortcomings of the prior art.

Another object of the present invention is to provide a process for obtaining chemically multi-functional nanomaterials.

Yet another object of the present invention is to provide a process to uniformly deposit multi-functional nanomaterials on solid surfaces of materials used in composite manufacturing.

Further an object of the present invention is to provide a composite material manufactured in the presence of chemically multi-functional nanomaterials as covalent nano-bridges at the interface between components.

### Summary of the Invention

A chemically multi-functional nanomaterial for use as binding agent in composite structures having at least two components chemically different from each other, wherein said chemically multi-functional nanomaterial is provided with a first functional group and with at least a second functional group different from said first functional group, is proposed. Also a method for production of said chemically multi-functional nanomaterials, wherein said method comprises reaction of an oxidized nanomaterial with a first compound and at least a second compound, wherein each of said compounds comprising at least two functional groups, at least one of said functional groups being carboxylic acid or hydroxyl reactive; and wherein at least one of said functional groups of said first compound is different than the functional groups of said second compound. Further, another method for production of said chemically multi-functional nanomaterials, wherein said method comprises reaction of an oxidized nanomaterial with a compound comprising at least three functional groups, at least one of said functional groups being carboxylic acid or hydroxyl reactive, and at least a second one of said functional groups is different than at least a third one of said functional groups.

Further, a composite structure comprising a first component, a second component connected to said first component, and said chemically multi-functional nanomaterials between said first component and said second component, wherein said first functional group is covalently bonded to said first component, and said at least second functional group is covalently bonded to said second component, is proposed.

### Brief Description of the Figures

The figures whose brief explanations are herewith provided are solely intended for providing a better understanding of the present invention and are as such not intended to define the scope of protection or the context in which said scope is to be interpreted in the absence of the description.
Figure 1 demonstrates the reaction of an oxidized nanomaterial according to the present invention, with at least two different multifunctional compounds comprising at least two functional moeities with at least one of them being carboxylic acid or hydroxyl reactive.
Figure 2 (a) and (b) represent two general schematic representations of symmetrically or asymmetrically poly-functional compounds, according to the present invention.
Figure 3 represents the reaction of an oxidized nanomaterial with a multi-functional compound possessing two or more different chemical functional groups and a carboxylic acid or hydroxyl reactive group, according to the present invention.
Figure 4 shows a general schematic representation of dendritic or branched poly-functional compounds prepared to produce chemically multi-functional nanomaterials according to the present invention.
Figure 5 demonstrates the reaction of an oxidized nanomaterial with a poly-functional compound to introduce alternative chemical functional groups and to increase the functionality of nanomaterials, which are then reacted with another multi-functional compound to introduce X and Y groups on said nanomaterial, according to the present invention.
Figure 6 shows a representative example of chemically multi-functional nanomaterials incorporated into a fiber reinforced polymeric composite structure according to the present invention.

### Detailed Description of the Invention

Referring now to the figures outlined above, the present invention proposes a method for obtaining chemically multi-functional nanomaterials being molecularly tailor-made for enhancing adhesion of surfaces of dissimilar materials to each other, for enhancing interfacial interactions in composite structures, for improving mechanical performance of composite structures and for introducing unique properties of nanomaterials into composite structures.

In other words, the present invention proposes chemically multi-functional nanomaterials for use as binding agents in composite structures, and a production method of said chemically multi-functional nanomaterials. Said chemically multi-functional nanomaterials are provided with a first functional group and at least a second functional group different from said first functional group.

A method according to the present invention is reacting an oxidized nanomaterial bearing carboxylic acid or hydroxyl moeities with two or more compounds different from each other and wherein each of said two or more compounds comprise at least two functional moeities, at least one of said functional moieties being carboxylic acid or hydroxyl reactive. An alternative method according to the present invention is reacting an oxidized nanomaterial with a multi-functional compound having at least two chemically functional groups different from each other, one of them being carboxylic acid or hydroxyl reactive, or further having an at least third carboxylic acid or hydroxyl reactive group. Said chemically multi-functional nanomaterials according to the present invention is suitable to be employed as binding agents of composite, layered composite or sandwich composite structures by joining surfaces of components made of following materials, wherein surfaces to be bound are made of materials different from each other such as polymeric materials, glass fiber, carbon fiber, aramid fiber natural fiber, metal, wood, plastic, glass or concrete. Further details of said multi-functional nanomaterials, a potential use thereof and alternative methods for preparing said chemically multi-functional nanomaterials according to the present invention are described below.

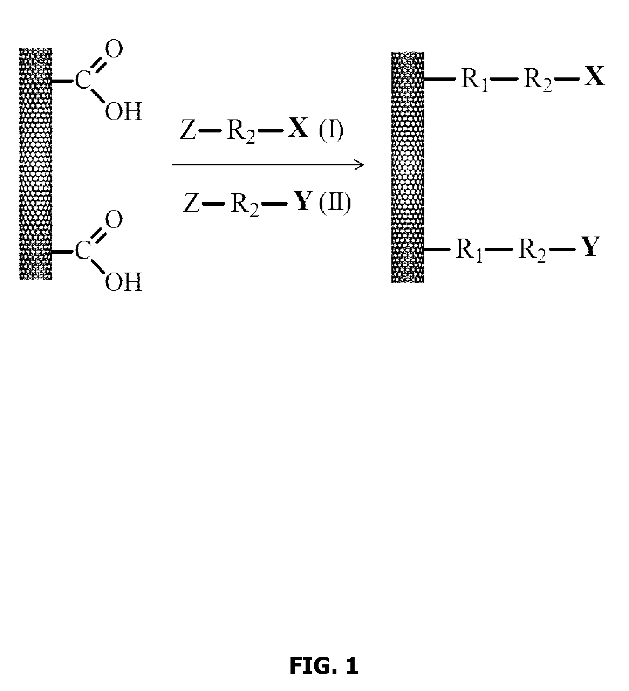

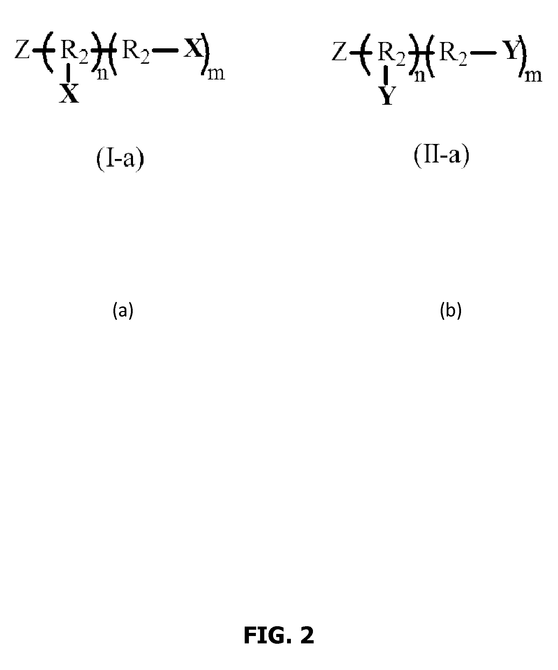

In a method according to the present invention, chemically multi-functional nanomaterials are prepared by first reacting carboxylic acid or hydroxyl moieties of oxidized nanomaterials with two or more different compounds comprising at least two functional moeities wherein at least one of them is carboxylic acid or hydroxyl reactive as shown in Figure 1. Oxidized nanomaterials can react with mixture of multifunctional compounds possessing at least two groups which are different from each other (said groups are represented as X and Y, and respective compounds comprising said groups are represented as I and II on Figure 1) and a carboxylic acid or hydroxyl reactive group at known ratios of said compounds I and II, or alternatively, subsequently by first reacting carboxylic acid or hydroxyl groups partially with compound I and then with compound II shown in Figure 1. Compounds I and II can be extended to various symmetrically or asymmetrically poly-functional compounds I-a and II-a with general schematic representation depicted in Figure 2; wherein if Z and X in compound I-a (or Y in compound II-b) are same, the compound is symmetrically poly-functional, and if Z and X in I-a (or Y in II-b) are different from each other, the compound is asymmetrically poly-functional.

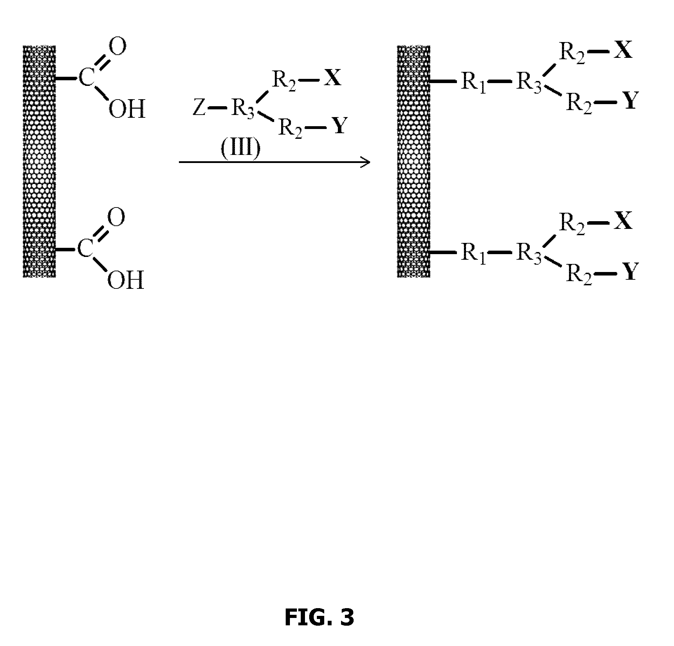

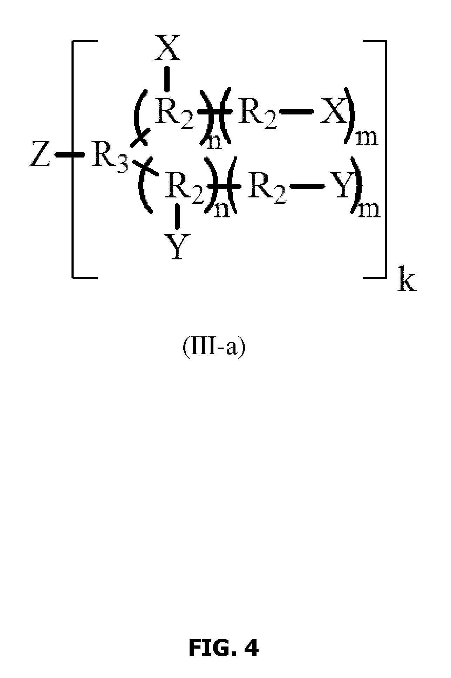

In an alternative method according to the present invention, chemically multi-functional nanomaterials are obtained by reacting an oxidized nanomaterial with a multi-functional compound represented as III in Figure 3, wherein said compound III comprises two or more chemically functional groups different from each other, represented as X and Y, and a carboxylic acid or hydroxyl reactive group on the same compound represented as Z. Compound III can be extended to dendritic or branched poly-functional compounds depicted in Figure 4 as compound III-a.

It is important to note that if Z and X or Z and Y are same in Figure 1, Figure 2, Figure 3, and Figure 4, special attention is needed when reacting oxidized nanomaterials with these compounds. In order to avoid undesired cross-linking between nanomaterials, preferably excess amount of these compounds are used such that the average number of carboxylic acid or hydroxyl reactive groups in these compounds is greater than, preferably at least twice the average number of carboxylic acid groups on nanomaterials.

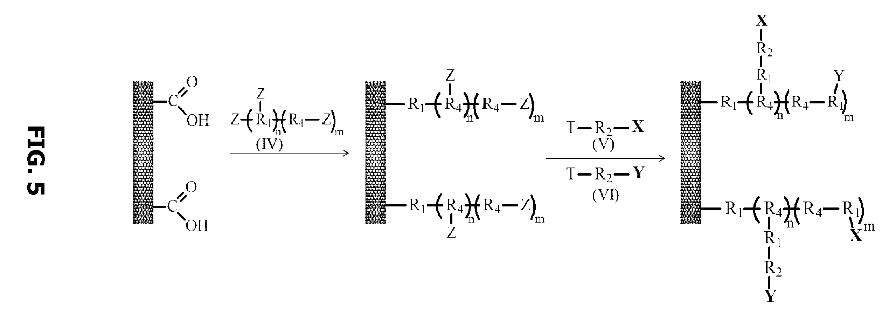

Alternatively, an oxidized nanomaterial can first be reacted with a poly-functional compound (represented as compound IV in Figure 5) to introduce alternative initial chemical functional groups to tailor and to increase the chemical functionality of nanomaterials, which are then reacted with further multi-functional compounds (represented as compounds V and VI having groups T, X and Y) to introduce groups X and Y on the same nanomaterial as shown in Figure 5.

Special attention is needed when reacting oxidized nanomaterials with compound IV in Figure 5. In order to avoid undesired cross-linking between nanomaterials, the average number of carboxylic acid or hydroxyl reactive groups (Z) in a reaction mixture is greater than, preferably at least twice the average number of carboxylic acid groups on nanomaterials. If T and X or T and Y are same in Figure 5, in order to avoid undesired cross-linking between nanomaterials, preferably excess amount of these compounds is used in the reaction mixture such that the average number of functional groups that can react with Z groups in the reaction mixture is greater than, preferably at least twice the average number of Z groups on nanomaterials.

According to the present invention, in compounds shown in Figures 1, 2, 3, 4 and 5, chemical functional groups depicted as Z can be any type of carboxylic acid or hydroxyl reactive group such as isocyanate, hydroxyl, amine, oxazoline, carbodimide or thiol as carboxylic acid reactive, isocyanate, anhydride or acyl halide for hydroxyl reactive groups.

According to the present invention, chemical functional groups depicted as X and Y which are not same with each other, is preferably chosen from any type of reactive organic functional groups including but not limited to isocyanate, blocked isocyanate, silane, ethylenically unsaturated double bond, thiol, hydroxyl, amine, acyl halide, alkyl halide, glycidyl ether, phenol, nitrile, cyanide, alkoxide, anhydride, caprolactam, oxazoline, carbodiimide and imine groups.

According to the present invention, R₁ groups are organic linkages preferably comprising ether, ester, urethane, urea, amide or imide; formed by the reaction of carboxylic acid or hydroxyl groups with functional groups represented as Z. According to the present invention, groups R₂, R₃ and R₄ preferably include alkyl, aromatic, branched alkyl, branched aromatic, oligomeric structures of known polymers that preferably comprise, polyethers, polyesters, polyurethanes, polyureas, epoxy, polyacrylates, polystyrene, acrylic copolymers, polysulfone, poly(ether sulfone), polyamide, polyimide, polyethylene, polypropylene, polyacrylonitrile, polycarbonate, polycaprolactone, polylactic acid and polybutadiene copolymer with molecular weights ranging from 100 to 20000 g/mol, more preferably 200 to 10000 g/mol or a mixture thereof.

In Figure 5, groups T according to the present invention, are chosen depending on chemical structures of groups Z, such that if Z is an isocyanate, T is preferably an amine or a hydroxyl; if Z is an amine, T is preferably an isocyanate, an acyl halide or glycidyl ether; and if Z is a hydroxyl, T is preferably an isocyanate, anhydride or acyl halide. In Figures 2, 4 and 5, n is greater or equal to 0 and m is greater or equal to 1.

Chemically multi-functional nanomaterials according to the present invention are extremely suitable to be employed in fiber-reinforced polymeric composites (FRPCs). Said chemically multi-functional nanomaterials can be based on carbon nanomaterials, other nanomaterials structurally similar such as graphitic boron nitride and boron nitride nanotubes, which can be chemically functionalized in the same manner as presented in the invention, and a mixture thereof. Said multi-functional nanomaterial is preferably based on a carbon nanomaterial selected from the list consisting of single-walled carbon nanotubes, double-walled carbon nanotubes, multi-walled carbon nanotubes, graphene, fullerene, graphite, carbon nanofiber and a mixture thereof. In graphite, graphene or few layer graphene exposed outer surfaces and edges will be decorated with the intended functionalities.

If said nanomaterials are obtained in their pristine form, then they are to be oxidized by known methods prior to application of the method according to the present invention.

In a preferred embodiment according to the present invention, said oxidized nanomaterials can be reacted with thionyl chloride first, then functionalization reactions explained above are carried out, in order to introduce more reactive acyl chloride groups on carbon nanomaterials in place of carboxylic acid groups.

Said chemically multi-functional nanomaterials according to the present invention comprise at least two (i.e. multiple) different chemically functional groups bonded thereon: at least a first one of said functional groups is for covalently bonding said nanomaterial to a surface of a first component; and at least a second one of said functional groups is for covalently bonding said nanomaterial to a surface of a second component constituting a composite structure.

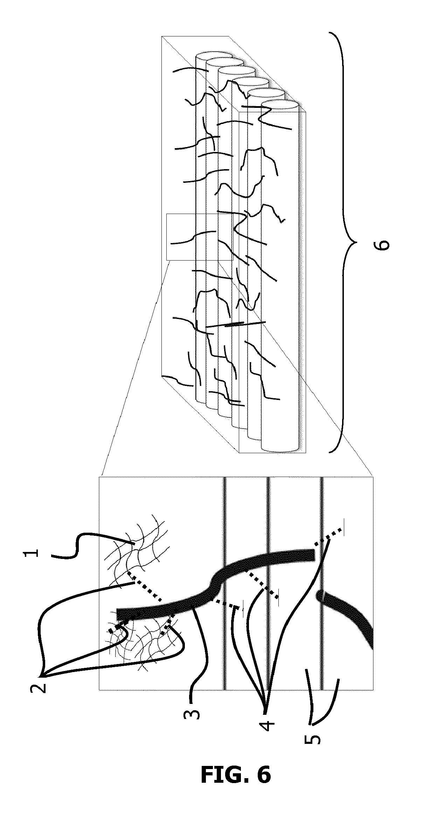

A representative example of chemically multi-functional nanomaterials incorporated into a fiber-reinforced composite structure according to the present invention is shown in Figure 6. In this figure, (1) is said first component (e.g. a thermoset polymer matrix formed by cross-linking); (2) is covalent bonding between said chemically multi-functional nanomaterial and the polymer matrix; (3) is a chemically multi-functional nanomaterial according to the present invention; (4) is covalent bonding between said chemically multi-functional nanomaterial and said second component; (5) is said second component; and (6) is a composite structure comprising chemically multi-functional nanomaterials according to the present invention.

In such composite structure, each component is expected to possess residual chemical functional groups on its surface or expected to be formed by chemical reactions, thus has a chemically reactive precursor during composite production.. Components of such composites that form by chemical reactions, thus have a chemically reactive precursor preferably comprise thermoset resins such as epoxy, polyurethane, polyester, vinyl ester, natural rubber, synthetic rubber, silicone rubber, resorcinol-formaldehyde, melamine, urea-formaldehyde, polyamide, polyimide, phenolic resin or a mixture thereof. Components comprising residual chemical functional groups preferably comprise materials selected from the list consisting of thermoplastic polymeric materials, thermoset polymeric materials, glass fiber, carbon fiber, aramid fiber, natural fiber, metal, wood, plastic, glass, concrete or a mixture thereof.

Such residual functional groups preferably include hydroxyl, carboxylic acid, amine, which are easily determinable by spectroscopic techniques such that said functional groups X and Y on a multi-functional nanomaterial is to be tailored according to the functionality type of each said component. If needed, solid surfaces can be treated chemically, thermally, by corona-discharge or by plasma to introduce said residual functional groups. In addition, said groups R₂, R₃ and R₄ are to be tailored to possess oligomeric chains to promote adhesion of each substrate physically to assist chemical bonding if required. A desired reaction for obtainment of chemical bonding between multi-functional nanomaterial and each component of said composite structure according to the present invention can occur at room temperature or thermal treatment may be required for such reactions. Upon said chemical bonding of the nanomaterial to each component of said composite at an interface between said components of said composite, mechanical strength of said composite is expected to be improved. In addition, unique mechanical, electrical and thermal properties of such nanomaterials are expected to be imparted.

According to the present invention, said nanomaterials can be applied onto a surface of said first component with the aid of conventional coating techniques such as spraying, brush coating, roll coating, solution casting or dip coating using a mixture of said nanomaterials in a liquid solvent. Said mixture comprising carbon nanomaterials can be prepared using known methods including high shear mixing or ultra-sonication in water or other common solvents including organic solvents such as dimethylformamide, tetrahydrofuran, acetone, methyl ethyl ketone, n-methylpyrrolidone, dimethyl sulfoxide, dimethyl acetamide, chloroform, dichloromethane, hexane or a mixture thereof. Said nanomaterials in said mixture are preferably applied onto a surface of said first component by electro-spraying, and a first type of abovementioned functional groups on said nanomaterials preferably readily react to form covalent bonds with said component's surface when first electro-sprayed or upon thermal treatment. Subsequently, said second component is introduced and at least a second (or further) type of abovementioned functional groups which are bonded on nanomaterials further react with said second component upon the addition of the second component followed by a subsequent thermal curing process if required.

In a fiber-reinforced polymeric composite with chemically multi-functional nanomaterials according to the present invention, for reactions of said chemically multi-functional nanomaterials with carbon, glass or aramid fiber surface, said chemically multi-functional nanomaterials preferably possess silane or isocyanate functionalities. As a second functionality, said chemically multi-functional nanomaterial to react with thermoset resins, e.g. with an epoxy thermoset resin, said chemically multi-functional nanomaterials preferably possess blocked isocyanate functionality, to react with hydroxyl groups generated on said epoxy matrix upon curing, amine or glycidyl ether functionalities to react with epoxy or amine groups of said thermoset resin, respectively. A vinyl, an acrylate or a methacrylate group functionality (having ethylenically unsaturated double bond) is preferred for a vinyl ester or rubber based system. An isocyanate, blocked isocyanate, alcohol, or amine can be incorporated for an epoxy, polyurethane, polyurea or poly(urethane urea) type of resin.

In a preferred embodiment of the composite structure according to the present invention, material comprised by said first component and material comprised by said second component are different from each other.

In order to obtain a composite structure provided with chemically multi-functional nanomaterials according to the present invention, an electrospray set-up is preferably integrated with composite manufacturing processes such as RTM, vacuum-assisted RTM (VA-RTM), vacuum infusion, pre-preg and hand lay-up.

Said electrospray set-up according to the present invention comprises a router which moves in x, y, and z directions, x and y being perpendicular to each other in a plane, and z being out-of-plane direction, with the aid of a computerized motor wherein the motorized or manual z-direction motion controls the height of a syringe pump in operation to electro-spray chemically multi-functional nanomaterials from a mixture onto the targeted surface. An advantage of the proposed electrospray process is that said chemically multi-functional nanomaterials according to the present invention can be homogeneously and individually dispersed on a targeted surface (namely, onto a surface of a first component) with tunable electric voltage resulting in minimized agglomeration. Another advantage of this process is that said process works complementarily to said chemically multi-functional nanomaterial structures and relevant industrial composite manufacturing processes, by depositing said chemically multi-functional nanomaterials on said targeted surface in a controlled manner. A further advantage of said process is that said chemically multi-functional nanomaterials are distributed on said targeted surface isotropically, which is ideal for functionalized carbon nanomaterials, since the functional groups are expected to be randomly located on said targeted surface. Yet another advantage of said process is that there are fewer occurrences of carbon nanomaterial agglomeration and resin infiltration during FRPC composite manufacturing process compared to pre-dispersion of carbon nanomaterials in resins in RTM or vacuum infusion processes.

## Claims

1. A chemically multi-functional nanomaterial for use as binding agent in composite structures having at least two components chemically different from each other, **characterized in that** said chemically multi-functional nanomaterial is provided with a first functional group and with at least a second functional group different from said first functional group.

2. A chemically multi-functional nanomaterial according to the Claim 1, wherein said chemically multi-functional nanomaterial is based on carbon a nanomaterial selected from the list consisting of single-walled carbon nanotubes, double-walled carbon nanotubes, multi-walled carbon nanotubes, graphene, fullerene, graphite, carbon nanofiber, graphitic boron nitride, boron nitride nanotubes or a mixture thereof.

3. A chemically multi-functional nanomaterial according to the Claim 1, wherein said first functional group and said second functional group are selected from the list consisting of isocyanate, blocked isocyanate, silane, ethylenically unsaturated double bond, thiol, hydroxyl, amine, acyl halide, alkyl halide, glycidyl ether, phenol, nitrile, cyanide, alkoxide, anhydride, caprolactam, oxazoline, carbodiimide and imine groups.

4. A chemically multi-functional nanomaterial according to the Claim 1, wherein said chemically multi-functional nanomaterial comprises one or more poly-functional organic structure selected from the list consisting of alkyl, aromatic, branched alkyl and branched aromatic structures, and oligomeric structure of polyether, polyester, polyurethane, polyurea, epoxy, polyacrylate, polystyrene, acrylic copolymers, polysulfone, poly(ether sulfone), polyamide, polyimide, polyethylene polypropylene, polyacrylonitrile, polycarbonate, polycaprolactone, polylactic acid and polybutadiene copolymer with molecular weights ranging from 100 to 20000 g/mol, or a mixture thereof.

5. A composite structure comprising a first component, a second component connected to said first component, and chemically multi-functional nanomaterials according to any of the previous claims between said first component and said second component, wherein said first functional group is covalently bonded to said first component, and said at least second functional group is covalently bonded to said second component.

6. A composite structure according to the Claim 5, wherein at least one of said first component or said second component comprises a thermoset resin selected from the list consisting of epoxy, polyurethane, polyester, vinyl ester, natural rubber, synthetic rubber, silicone rubber, resorcinol-formaldehyde, melamine, urea-formaldehyde, polyamide, polyimide, phenolic resin or a mixture thereof.

7. A composite structure according to the Claim 6, wherein at least one of said first component or said second component comprises residual chemical functional groups, and wherein said component comprising said chemical functional groups further comprises material selected from the list consisting of thermoplastic polymeric materials, glass fiber, carbon fiber, aramid fiber, natural fiber, metal, wood, plastic, glass, concrete or a mixture thereof.

8. A composite structure according to the Claim 6 or Claim 7, wherein said first component or said second component comprises material selected from the list consisting of thermoplastic polymeric materials, said thermoset resins, glass fiber, carbon fiber, aramid fiber, natural fiber, metal, wood, plastic, glass, concrete or a mixture thereof.

9. A composite structure according to the Claim 7 or Claim 8, wherein material comprised by said first component and material comprised by said second component are different from each other.

10. A method of production of chemically multi-functional nanomaterials for use as binding agent in composite structures having at least two components chemically different from each other, wherein said chemically multi-functional nanomaterial is provided with a first functional group and at least a second functional group different from said first functional group, **characterized in that** said method comprises
- reaction of an oxidized nanomaterial with a first compound and at least a second compound, wherein each of said compounds comprising at least two functional groups, at least one of said functional groups being carboxylic acid or hydroxyl reactive, and wherein at least one of said functional groups of said first compound is different than the functional groups of said second compound.

11. A method of production of chemically multi-functional nanomaterials for use as binding agent in composite structures having at least two components chemically different from each other, wherein said chemically multi-functional nanomaterial is provided with a first functional group and at least a second functional group different from said first functional group, **characterized in that** said method comprises
- reaction of an oxidized nanomaterial with a compound comprising at least three functional groups, at least one of said functional groups being carboxylic acid or hydroxyl reactive, and at least a second one of said functional groups is different than at least a third one of said functional groups.

12. A method according to the Claims 11 or 12, wherein at least one of said compounds is a polyfunctional compound having a molecular weight ranging from 100 g/mol to 20000 g/mol.

13. A method according to the Claim 13, wherein said polyfunctional compound has a molecular weight ranging from 200 g/mol to 10000 g/mol.
